# EUROPEAN PATENT APPLICATION

(11) **EP 2 716 303 A1**
(43) Date of publication of application: **09.04.2014**
(21) Application number: 12791976.9
(22) Date of filing: 29.05.2012
(51) Int. Cl.: A61K 45/00, A23L 1/22, A23L 1/221, A23L 1/226, A23L 1/30, A61K 8/42, A61K 8/49, A61K 8/97, A61K 31/16, A61K 31/164, A61K 31/36, A61K 31/4525, A61K 36/18, A61K 36/28, A61K 36/75, A61P 25/26, A61Q 11/00, A61Q 13/00, A61Q 19/00, C11B 9/00

(54) **PSYCHIC ENERGIZER AGENT AND PSYCHIC ENERGIZER COMPOSITION**

(30) Priority: 31.05.2011 JP 2011121650
(71) Applicant: Takasago International Corporation, Tokyo-to 144-8721 (JP)
(72) Inventor: TAKAHASHI Kaori, Hiratsuka-shi Kanagawa 254-0073 (JP); TAKAYANAGI Miyuki, Hiratsuka-shi Kanagawa 254-0073 (JP)
(74) Representative: Uchida, Kenji
(86) International application number: PCT/JP2012/063705
(87) International publication number: WO 2012/165406

(57) **Abstract**

The present invention relates to a stimulating agent comprising a pungent component having astringency, a stimulating composition including the agent, as well as a flavor or fragrance composition, a food, a drink, an oral care product and an external skin preparation containing the agent or the composition, and also relates to the use of a pungent component having astringency as a stimulating agent. Examples of the pungent component having astringency include a plant extract of Asteraceae Spilanthes, Rutaceae Zanthoxylum, Rutaceae Fagara, or Piperaceae Piper, or an amide derivative represented by general formula (1) (where R¹ represents an alkenyl group having 2 to 12 carbon atoms which may be substituted by a methylenedioxyphenyl at the end, R² and R³ may be the same or different from each other and each are a hydrogen atom or a lower alkyl group in which a hydroxyl group may be substituted, and R² and R³, together with the nitrogen atom adjacent thereto, may form a heterocyclic ring).

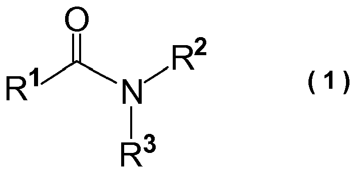

## Description

### Technical Field

The present invention relates to a stimulating agent and stimulating compositions, which have an effect of exalting spirits; a fragrance composition, a food, a drink, an oral composition and an external skin preparation, which contain the stimulating agent or the stimulating composition; and use of a compound or a composition as a stimulating agent.

### Background Art

A need for living a fulfilling life in body and mind has been increasing in recent years. With this need, researches made progress in various fields and useful substances have been made into products. In the present day wherein there are strong interests for stress relaxation and mood altering, great interests in substances having an effect uplifting or calming the arousal level of humans have risen.

As a method for examining about an effect uplifting or calming the arousal level of humans, there is a method of indexing gradual potential changes in human brain (Contingent Negative Variation: CNV). CNV relates to changes of mental processes and arousal levels such as attention, expectation, and anticipation. It is known that when an arousal level is high, an early component of CNV increases compared to normal state and when an arousal level is low, an early component of CNV decreases. For example, it was reported that when the subjects smelled the odour of jasmine oil, which is known empirically to have stimulant effect, an early component of CNV increased whereas when subject smelled a lavender type fragrance Lavande, an early component of CNV decreased (for example, see Torii et al., 19th Symposium of Taste and Smell, September 11, 1985). Lavender has been known as a material having a calming effect. Similarly, materials which have a stimulant effect have been searched hitherto, and natural essential oils or synthetic aroma chemicals consisting of single compounds, which show a stimulative effect by smelling the fragrance thereof, were found (Japanese Patent Application Laid-Open No. S63-199293). Further, it is known that a particular ingredient contained in essential oils and a composition containing the ingredient have a stimulative effect (Japanese Patent Application Laid-Open Nos. 2001-19992 and 2003-119491).

As described above, an aroma and fragrance uplifting the arousal level of humans are known but most of them conventionally known to stimulate minds by aspirating from the nasal cavity. Diversification of beverage containers such as a can and a PET bottle has proceeded with the change of eating habits and a chance of direct drinking from a vessel without transferring to a glass has been increased. Therefore, development of ingredients which bring about an effect uplifting the arousal level through flavor or stimulus of an oral cavity by eating and drinking or through skin stimulus etc. are required.

The present invention has been made in view of such situations as mentioned above and an object of the invention is to provide a new stimulating agent which brings about an effect uplifting the arousal level of humans through the flavor or stimulus of an oral cavity by eating or drinking or through the skin stimulus. Further, another object of the present invention is to provide a new composition for stimulating the nervous system and a flavor or fragrance composition, a food, a drink, an oral composition, and an external skin preparation containing a new stimulating agent or stimulating composition. Furthermore, another object of the present invention relates to use of a compound or a composition as a stimulating agent.

The present inventors have eagerly studied about compounds or compositions having a stimulative effect. As a result, the present inventors found that a pungent component having astringency and a composition containing a pungent component having astringency as an effective ingredient provide the stimulative effect of mind (psychic stimulative effect) through stimulus of an oral cavity or a skin and the present invention was made based on these findings.

### Summary of the Invention

The present invention relates to a stimulating agent, a stimulating composition, and a flavor or fragrance composition, a food, a drink, an oral composition and an topical agent containing the stimulating agent or the stimulating composition, as well as use of a compound or a composition as a stimulating agent as described below.
(1) A stimulating agent consisting of a pungent component having astringency.
(2) The stimulating agent described in the above item (1), which is characterized in that the pungent component having astringency is an extract of one or two or more of plants belonging to a genus selected from a group consisting of the genus Spilanthes of family Asteraceae, the genus Zanthoxylum of family Rutaceae, the genus Fagara of family Rutaceae, and the genus Piper of family Piperaceae.
(3) The stimulating agent described in the above item (1), which is characterized in that the pungent component having astringency is an amide derivative represented by the following formula (1) : 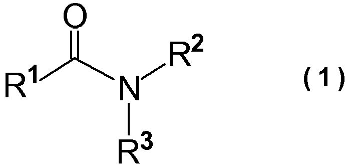 wherein R¹ represents an alkenyl group having 2 to 12 carbon atoms which may be substituted by a methylenedioxyphenyl group at the end, R² and R³ may be the same or different from each other and each represents a hydrogen atom or a lower alkyl group which may be substituted by a hydroxyl group, and R² and R³ may form a heterocyclic ring together with a nitrogen atom adj acent thereto.
(4) The stimulating agent described in the above item (3), which is characterized in that the amide derivative represented by the formula (1) is one or two or more of amide derivatives selected from a group consisting of spilanthols, sanshools, hydroxy-sanshools, and piperines.
(5) A stimulating composition containing a pungent component having astringency as an effective ingredient.
(6) The stimulating composition described in the above item (5), which is characterized in that the pungent component having astringency is an extract of one or two or more of plants belonging to a genus selected from a group consisting of the genus Spilanthes of family Asteraceae, the genus Zanthoxylum of family Rutaceae, the genus Fagara of family Rutaceae, and the genus Piper of family Piperaceae.
(7) The stimulating composition described in the above item (5), which is characterized in that the pungent component having astringency is an amide derivative represented by the following formula (1) : 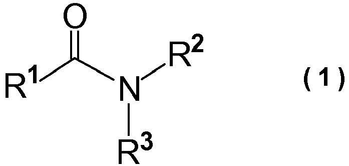 wherein R¹ represents an alkenyl group having 2 to 12 carbon atoms which may be substituted by a methylenedioxyphenyl group at the end, R² and R³ may be the same or different from each other and each represents a hydrogen atom or a lower alkyl group which may be substituted by a hydroxyl group, and R² and R³ may form a heterocyclic ring together with a nitrogen atom adj acent thereto.
(8) The stimulating composition described in the above item (7), which is characterized in that the amide derivative represented by the formula (1) is one or two or more of amide derivatives selected from a group consisting of spilanthols, sanshools, hydroxy-sanshools, and piperines.
(9) The stimulating composition described in any one of above items (5) to (8), which is characterized in that the pungent component having astringency is contained in 0.000001 to 10.0 % by mass.
(10) A flavor or fragrance composition which is characterized in that the stimulating agent described in any one of the above items (1) to (4) or the stimulating composition described in any one of the above items (5) to (9) is contained.
(11) A food, a drink, an oral composition or an external skin preparation which is characterized in that the stimulating agent described in any one of the above items (1) to (4) or the stimulating composition described in any one of the above items (5) to (9) is contained.
(12) A food or drink, an oral composition or an external skin preparation which is characterized in that the flavor or fragrance composition described in the above item (10) is contained.
(13) Use of a pungent component having astringency as a stimulating agent.
(14) Use described in the item (13) which is characterized in that the pungent component having astringency is an extract of one or two or more of plants belonging to a genus selected from a group consisting of the genus Spilanthes of family Asteraceae, the genus Zanthoxylum of family Rutaceae, the genus Fagara of family Rutaceae, and the genus Piper of family Piperaceae.
(15) Use described in the item (13) which is characterized in that the pungent component having astringency is an amide derivative represented by the following formula (1): 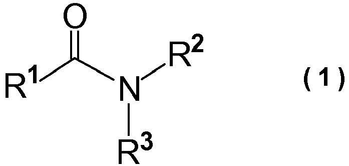 wherein R¹ represents an alkenyl group having 2 to 12 carbon atoms which may be substituted by a methylenedioxyphenyl group at the end, R² and R³ may be the same or different from each other and each represents a hydrogen atom or a lower alkyl group which may be substituted by a hydroxyl group, and R² and R³ may form a heterocyclic ring together with a nitrogen atom adj acent thereto.
(16) Use described in the item (15) which is characterized in that the amide derivative represented by the formula (1) is one or two or more of amide derivatives selected from a group consisting of spilanthols, sanshools, hydroxy-sanshools, and piperines.

### Advantageous Effect of the Invention

In the present invention, a stimulative effect is provided by adding a stimulating agent or a stimulating composition to a flavor or fragrance composition, a food, a drink, an oral composition, and an external skin preparation as following modes. That is, in the case of the food or drink, the stimulative effect is obtained through stimulus of an oral cavity or skin by eating or drinking. Further, in the case of the flavor or fragrance composition, the stimulative effect is obtained through stimulus of an oral cavity or skin by eating or drinking a food or drink to which the flavor or fragrance composition is added. Furthermore, in the case of the oral composition, the stimulative effect is obtained through stimulus of an oral cavity or skin by taking it into a mouth or after spiting it out. Further, in the case of the external skin preparation, the stimulative effect is obtained through skin stimulus by attaching it to the skin.

### Brief Description of the Drawings

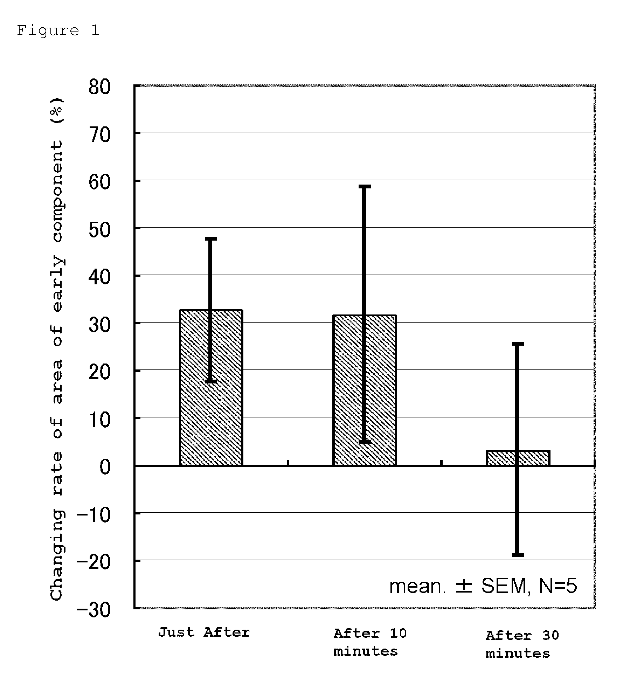

Figure 1 shows the results of CNV measurement when 100 ml of 30 ppm aqueous solution of jambu extract which is a stimulating agent was taken into a mouth (Example 1). The vertical axis represents the area (unit: msec. x µV) of the early component of CNV between 400 and 1, 000 milliseconds after giving a warning sound stimulus (S1) in percentage (%) when the area of the early component before intaking a sample was set to 100 %. In Figure 1, each of bold lines shows the mean value of the measurement results and the upward shift shows a stimulation state and the downward shift shows a sedation state compared to normal state. Further, each of thin lines shows a standard error of the mean (SEM) .

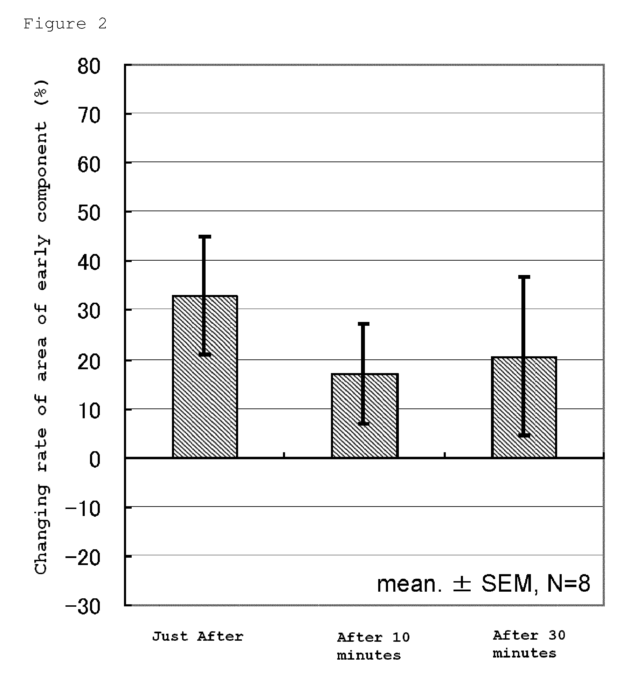

Figure 2 shows the results of CNV measurement when 100 ml of 75 ppm aqueous solution of Japanese pepper extract which is a stimulating agent was taken into a mouth (Example 2). The vertical axis expresses the area (unit: msec. x µV) of the early component of CNV between 400 and 1, 000 milliseconds after giving a warning sound stimulus (S1) in percentage (%) when the area of the early component of CNV before intaking a sample was set to 100 %. In Figure 2, each of bold lines shows the mean value of the measurement results and the upward shift shows a stimulation state and the downward shift shows a sedation state compared to normal state. Further, each of thin lines shows a standard error of the mean (SEM).

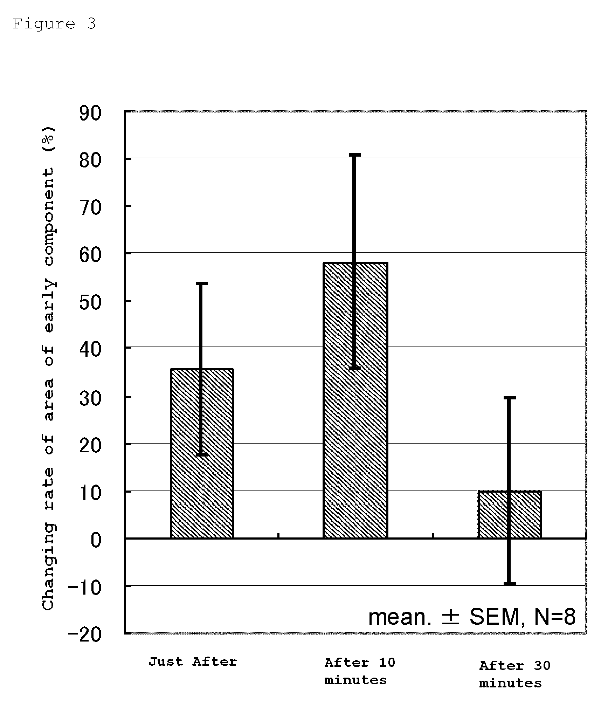

Figure 3 shows results of CNV measurement when 100 ml of 50 ppm aqueous solution of white pepper oleoresin which is a stimulating agent was taken into a mouth (Example 3). The vertical axis expresses the area (unit: msec. x µV) of the early component of CNV between 400 and 1, 000 milliseconds after giving a warning sound stimulus (S1) in percentage (%) when the area of the early component before intaking a sample was set to 100 %. In Figure 3, each of bold lines shows the mean value of the measurement results and the upward shift shows a stimulation state and the downward shift shows a sedation state compared to normal state. Further, each of thin lines shows a standard error of the mean (SEM).

### Form for Carrying out the Invention

Hereinafter, the stimulating agent and the stimulating composition of the present invention as well as the flavor or fragrance composition, the food, the drink, the oral composition and the external skin preparation containing the stimulating agent or the stimulating composition of the present invention will be described in detail in order.

First, in the present invention, a stimulating agent and a stimulating composition mean an agent or a composition which exerts psychologically stimulant feeling to users when used. Further, a pungent component having astringency means a compound having astringency in compounds known as a content having a pungent taste. In the present invention, the pungent taste means a taste having an action causing pain feeling and thermal sensing (stimulation feeling) and the astringency means a combined sensation like sensations of straining or contracting the oral mucous membrane and the skin and puckering up the mouth. The pungent component having astringency consists of at least one kind of compounds showing astringency and a pungent taste and it may consist of multiple compounds showing astringency and a pungent taste.

The stimulating agent and the stimulating composition of the present invention give a stimulative effect to a food, a drink, an oral composition such as toothpaste and mouthwash, an external skin preparation such as cosmetics, antiperspirants, and the like by containing it in these products. The stimulative effect means an effect which can release humans from bad physiological mental state such as depression and uneasiness experiencing in daily life, raising a feeling, and activating mental activity. The effect can be confirmed by a sensory evaluation using a panel or an index being capable of detecting a stimulant feeling, for example CNV measurement etc.

Many compounds such as sanshool, piperine, spilanthol, fagaramide, and so on are known as a pungent component having astringency. Spilanthol is an ingredient contained in, for example, S. acmella var. oleracea and S. acmella, which are annual plants of Asteraceae Spilanthes native to South America and called jambu in Brazil, and the like. Sanshools are ingredients contained in Rutaceae Zanthoxylum or Rutaceae Fagara, for example Zanthoxylum piperitum, Fagara schinifolium, and the like. Fagaramides are ingredients contained in Fagara schinifolium and the like. Piperines are ingredients contained in Piperaceae Piper, for example Piper nigrum, Piper longum, Piper retrofractum, and the like.

The compounds constituting a pungent component having astringency may be compounds which are synthesized and may be natural products, for example, extracts obtained by extracting from a plant containing a pungent component having astringency in the present invention.

Amide derivatives represented by the formula (1) described above are mentioned as the example of pungent component having astringency which is a component of the stimulating agent and stimulating composition of the present invention. In the formula (1) of the amide derivatives, R¹ represents an alkenyl group having 2 to 12 carbon atoms which may be substituted by a methylenedioxyphenyl group at the end. Concrete examples thereof include a vinyl group, a 1-butenyl group, a 1,3-butadienyl group, a 5-hexenyl group, a 1,5-hexadienyl group, a 1,3,5-hexatrienyl group, a 7-octenyl group, a 1,7-octadienyl group, a 1,3,7-octatrienyl group, a 1,5,7-nonatrienyl group, a 1,3,9-decatrienyl group, a 1,3,11-tridecatrienyl group, a 1,5,7,9-undecatetraenyl group, and so on. Further, in the formula (1) of the amide derivatives, R² and R³ may be the same or different from each other and each represent a hydrogen atom or a lower alkyl group which may be substituted by a hydroxyl group. In addition, R² and R³ may form a heterocyclic ring together with an adjacent nitrogen atom. Concrete examples of the lower alkyl group which may be substituted by a hydroxyl group include a methyl group, an ethyl group, an n-propyl group, an iso-propyl group, an n-butyl group, an iso-butyl group, a 2-hydroxy-2-methylpropyl group, and so on. A piperidinyl group and the like are mentioned as the example of the heterocyclic ring which is formed by R² and R³ together with a nitrogen atom adjacent thereto.

As the amide derivative represented by the formula (1), which is a pungent component having astringency, there are typically exemplified sanshools, hydroxy-sanshools, piperines, spilanthols, and fagaramide as described above.

Examples of sanshools include, for example, α-sanshool, and β-sanshool which are represented by the following formulae, γ-sanshool, δ-sanshool, and the like.

### α-Sanshool:

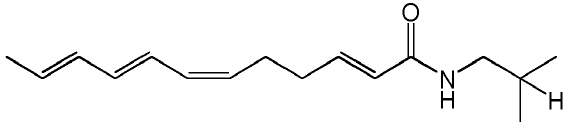

### β-Sanshool:

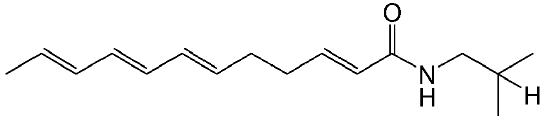

As hydroxy-sanshools, there are exemplified hydroxy-α-sanshool and hydroxy-β-sanshool which are represented by the following formulae, hydroxy-γ-sanshool, hydroxy-δ-sanshool and the like.

### Hydroxy-α-sanshool:

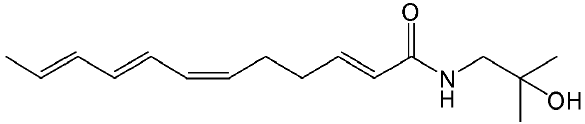

### Hydroxy-β-sanshool:

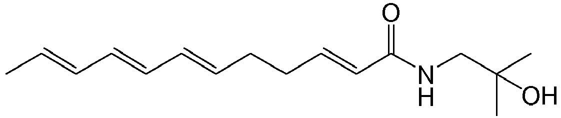

As spilanthols, there are exemplified spilanthol having a structure described below, homospilanthol, and the like.

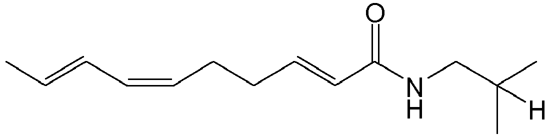

Piperines are compounds in which a piperidine and piperic acid are bonded by an amide bond and have a following structure.

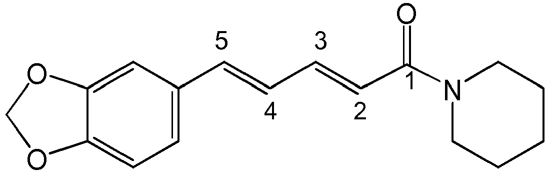

In the structure described above, a (2,3)-trans and (4,5) -trans form is piperine, a (2,3)-cis and (4,5) -trans form is isopiperine, a (2, 3) -trans and (4, 5) -cis form is isochavicine, and a (2,3)-cis and (4,5)-cis form is chavicine. Further, piperiline which is a compound wherein piperidine is changed to pyrrolidine in the aforementioned structure is also mentioned as the examples of Piperines.

Fagaramide has a following structure.

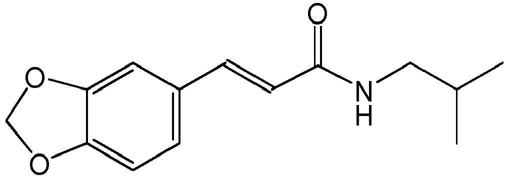

As extracts in which a pungent component having astringency is obtained by extraction from a plant containing a pungent component having astringency, there are exemplified extracts obtained by extraction from Asteraceae Spilanthes containing spilanthols, Rutaceae Zanthoxylum and Asteraceae Fagara containing sanshools, Piperaceae Piper containing piperines, and so on. These are available as jambu extract, jambu oleoresin, sansho extract, sansho essential oil, white pepper oleoresin, white pepper essential oil, black pepper oleoresin, black pepper essential oil, and so on.

An extraction method of an extract of a plant containing a pungent component having astringency may be any known methods. For example, jambu extract is obtained by extracting a dried product of whole S. acmella var. oleracea or S. acmella or a dried product of a flower part thereof, which are used as it is or after powdered, with organic solvent such as alcohol, acetone, hexane and the like, removing solvent from the extract, distilling the thus obtained concentrate, and being treated by a column. Spilanthol concentration thereof is about 80 %.

The stimulating agent of the present invention can provide a stimulative effect as inclusion of food products, drink products, oral compositions such as toothpaste and an oral refrigerant, external skin preparations such as cosmetics and an antiperspirant, and the like. As the stimulating agent, these may be used a chemical compounds known as a pungent component having astringency itself, an extract itself or a solution thereof dissolved in solvent. Examples of the solvent include ethanol, propylene glycol (PG), dipropylene glycol (DPG), benzyl benzoate, water, triacetin, triethyl citrate, and medium chain fatty acid triglyceride (MCT).

In the present invension, the pungent component having astringency can be also used in a variety of forms. The products are in various shapes prepared by processing the pungent component having astringency such as powder, granulation, and emulsion. The forms are not particularly limited if it is one of the forms commonly known. Any one of commonly practiced processing methods such as powdering, granulation, and emulsification may be suitably used as the form processing method.

As the powdering methods, there are exemplified an adsorption powdering method wherein the pungent component having astringency is adsorbed on saccharides such as oligosaccharide, dextrin, or starch, which are used as an excipient (carrier), and a method of spray-drying the pungent component having astringency together with the above-described excipient and an emulsifier such as a fatty acid ester of sucrose, a fatty acid ester of polyglycerols, and quillaj a saponin. Further, a method of spray-drying the pungent component having astringency with a natural gum such as gum arabic, a processed starch, or the like is also raised. Yet there are other methods including an extrusion-molding method of mixing the pungent component having astringency with two or more of saccharides such as sucrose and maltose, and sugar alcohols such as palatinit and maltitol, then dissolving the mixture, for example, in water under heat, extruding, and drying to form powder; and a coacervation method utilizing the phase separation by gelatin, agar or the like. Any one of the powering methods described above can be adopted according to applications of the pungent component having astringency to prepare a powdery formed product.

In addition, the powder obtained by the aforementioned method may be further processed together with gelatin, pullulan or lactose as a binder by a fluidized-bed granulation method to prepare a product in a granular shape. The powder or granule obtained by the above-described method may be coated additionally. Any one of known methods can be used as the coating method and examples thereof include spray coating, fluidized-bed coating, centrifugal coating, and contact coating. As the coating materials, any one of water-soluble coating materials such as saccharides, pectin, agar, methylcellulose, pullulan, and gelatin and oil-soluble coating materials such as hydrogenated oils which are solid at a normal temperature, for example, rice bran wax and palm oil may be used suitably according to applications. In these methods, it is also possible to use a water-soluble coating material and an oil-soluble coating material in combination.

The products prepared by the emulsification may also be those prepared by any one of known methods. Examples thereof include an emulsion prepared by dissolving, for example, the amide derivative together with an emulsifier such as a fatty acid ester of sucrose, a fatty acid ester of polyglycerol, quillaja saponin, or lecithin or a natural gum such as gum arabic, in a solvent and then stirring and blending the solution, for example, in a TK mixer or a high-pressure homogenizer to emulsify.

In the present invention, the pungent component having astringency may be used alone as the stimulating agent. Further, a stimulating composition is prepared by compounding one or more kinds selected from other component providing stimulative effect; other component not providing stimulative effect such as flavors, coloring matters, acidulants, vitamins, sweeteners, seasonings, spices, food materials, and functional substances; and solvent to the aforementioned pungent component having astringency as needed and it may be used as a stimulating agent. It is necessary to contain the pungent component having astringency as an active ingredient in the stimulating composition of the present invention. The term 'contain as an active ingredient' means that the pungent component having astringency is contained in a sufficient amount providing a stimulative effect. The amount of the pungent component having astringency in the stimulating composition differs according to kinds of the pungent component having astringency used and it might not be regulated unconditionally by the use, character, and use method of products in which the stimulating composition of the present invention is compounded. It can be selected freely according to kinds of the object, kinds of flavor compounded, and the like within the limit to exhibit the stimulative effect.

These other components providing a stimulative effect, other components not providing a stimulative effect, and solvent can be utilized as a form of mixture mixed with a pungent component having astringency, a form of solution or formed products such as powder, granulation, and emulsion. The formed products can be formed by the same form and the same method as those described in the aforementioned formed products of component providing a stimulative effect.

As other components providing a stimulative effect, there are exemplified menthol, menthone, limonene, pinenes, linalool, geranial, citronellal, camphor, thymol, piperitone, isoamyl angelate, phenylethyl angelate, cumic alcohol, menthalactone, ethyl myristate, and perillaldehyde (see Japanese Patent Application Laid-Open No. 2010-65233); 2,2,6-trialkylcyclohexanecarboxylic acid esters and 2,2,6-trialkylcyclohexenecarboxylic acid esters (see WO 2005/049773 A1), anisaldehyde, cinnamicaldehyde, anethole, eugenol, carvone, heliotropine, mint oil, cinnamon oil, star anise oil, clove oil, caraway oil, pepper oil, cardamom oil, and nutmeg oil (see Japanese Patent Application Laid-Open No. 2001-19992) ; basil oil, cassia oil, jasmine oil, neroli oil, rose oil, ylang-ylang oil etc., which are raw materials of flavor or fragrance, or components of flavor or fragrance. These components may be used together with caffeine, capsaicin, spices, and so on. The stimulative effect of amide derivatives are enhanced by combining these materials.

Examples of the solvent used include the same solvents as those used for dissolving a pungent component having astringency, for example, ethanol, propylene glycol (PG), dipropylene glycol (DPG), benzyl benzoate, water, triacetin, triethyl citrate, medium chain fatty acid triglyceride (MCT) etc.

The stimulating agent and stimulating composition of the present invention can be made to a flavor or fragrance composition by containing suitably a flavor or fragrance component or an essential oil, which is generally used, within a limit not canceling the intended stimulative effect for improving palatability of scents and the like. Further, components which are able to be contained in a flavor or fragrance composition generally, for example, an antioxidant, an antiseptic, a chelating agent, an ultraviolet absorber, and a coloring matter may be contained in the flavor or fragrance composition.

Furthermore, the stimulating agent or stimulating composition of the present invention can provide a stimulative effect to an object, which is able to contain flavor or fragrance, by containing as a flavor or fragrance compound mainly. As the object, there are exemplified, but are not limited to, foods, drinks, oral compositions and external skin preparations.

The amount of the stimulating agent of the present invention to application objects such as a food, a drink, an oral composition, an external skin preparation, and the like differs by the uses and properties of the objects and cannot be regulated uncondituinally. However, it can be selected freely according to the kinds of objects or the kind of flavor or fragrance compounds within the limit capable of providing a stimulative effect. Usually, the amount of the pungent component having astringency is preferably from about 0.000001 % by mass to about 10.0 % by mass relative to a total mass of the product. If the amount thereof is less than 0.000001% by mass to the object, an insufficient stimulative effect tends to be provided even if the influence of other compounding components is kept to a minimum. On the other hand, if the amount thereof is more than 10.0% by mass, improvement of the stimulative effect suitable for the content of the component having a stimulative effect is not provided and the balance of odor tends to be unfavorable generally. The amount of the pungent component having astringency may vary in a wide range to various products and the amount of the stimulating composition and flavor or fragrance composition added to the products also varies by various conditions. It is, therefore, preferred that the amounts of the stimulating composition and the flavor or fragrance composition are the same amount as the aforementioned content of the pungent component having astringency. That is, it is preferred that these compositions are contained in an amount of 0.000001% by mass to 10.0% by mass relative to total mass of the product.

Examples of the foods or the drinks which are objects containing the stimulating agent, stimulating composition, or flavor composition of the present invention include drinks such as green tea, black tea, coffee, soft drink, soymilk, lactic fermented milk drink, fruit juice, vegetable juice, carbonated drink, liquor, and energy drink; confectionery products such as chocolate, candy, and chewing gum; frozen desserts such as ice cream, lollipop, sherbet, and shaved ice; deserts such as cake, cream, jelly, mousse, and pudding; and seasonings such as salad dressing, sauce and margarine.

Examples of the oral composition include toothpaste, tooth powder, oral wash, mouth wash, and so on. Examples of the external skin preparation include cosmetics such as soft lotion, astringency lotion, wipe-cleaning lotion, milky lotion, whole body lotion, after-shaving lotion, after-shaving gel, massage cream, cleansing cream and cleansing gel; hair cosmetics such as shampoo, hair conditioner, hair tonic, and hair cream; convenience goods such as antiperspirant and liquid or powder bathing agent; quasi drugs such as hair-growth lotion, ointment, and thermal sensation or cooling sensation cataplasm.

The target which intakes the stimulating agent of the present invention, the stimulating composition of the present invention or the flavor or fragrance composition containing these of the present invention through a mouth is usually human being but may be a mammal such as a dog and a cat. Intaking those through a mouth is conducted with foods, drinks, oral care products, quasi drugs such as external skin preparations, which contain the stimulating agent, the stimulating composition or the flavor composition containing these. That is, by intaking the stimulating agent, the stimulating composition or the flavor composition containing these through a mouth according to the usual usage, which is included in foods, drinks, oral care products, and quasi drugs, the minds of the targets (humans being and a mammal) are stimulated by the stimulating component.

### EXAMPLES

Hereinafter, the present invention will be described specifically with reference to examples. In addition, '%' in a blending quantity below means 'percent by mass' relative to an object to be blended.

### (CNV test method)

A stimulative effect is examined by measuring changes of a negative potential which is called as Contingent Negative Variation (CNV). The CNV is known as a slow change of potential in a brain which is interlocked with changes of mental processes and arousal levels such as attention, expectation, anticipation, and the like. It was reported that when humans took in caffeine which shows a stimulative effect, the amplitude of CNV increased whereas when humans absorbed Nitrazepam which shows a sedative effect, the amplitude of CNV declined. This CNV test method is well known as a measuring method of a stimulative effect as described in Japanese Patent Application Laid-Open Nos. S63-199292 and S63-199293.

The CNV measurement in the present invention was conducted under following conditions.

The CNV was measured by using a digital electroencephalograph, SYNAFIT 2500 manufactured by NEC Medical Systems Ltd. Electrodes for a CNV measurement were placed at Fz according to the international 10-20 system, electrodes for ear lobes were set to indifferent electrodes, and brain waves were recorded at a time constant of 5.0 seconds referenced to the linked earlobes. In order to check artifacts of eye movement to a brain wave, vertical movements of the left eye were also recorded.

Confirmation of a stimulative effect of a sample was conducted by steps of giving S1 (tone burst sound), giving S2 (light displayed on CRT) at 2.3 seconds after giving S1, and asking subjects movement reaction (MR) of pressing a button after giving S2. Control (blank) conditions were measured by intaking water instead of the sample.

The averaged evoked potential was obtained by adding 20 times or more trials after removing an artifact such as eye movement by a software EPLYZERII. The base line was determined from an average voltage of a brain wave in a time period of 500 msec. before S1. The evaluation of the CNV waveform was conducted according to a method of Torii et al., in which CNV was evaluated by the area (unit: msec. x µH) of an early component between 400 msec. to 1,000 msec. after a warning sound stimulus (S1). That is, in the case that the area of the CNV early component at a measurement after intaking a test sample (or water) increases or decreases in comparison with that at a measurement before intaking a test sample or water, it was judged as to be effective. The change ratio of before intaking condition to after intaking condution was shown in percentage (%), and the change ratio was made to an index of the stimulative effect. Positive number of the index suggests test sample has a stimulative effect, whereas negative number of the index suggests test sample has a calming effect.

### (Test subjects and test method)

In each intaking test, healthy 5 to 8 adults were adopted as test subjects. CNV measurements were conducted before, just after, 10 minutes after, and 30 minutes after intaking a test sample or a control sample according to a procedure described above. Change ratio of the CNV area at each session after intaking the test sample or the control to the session before intaking the test sample or the control was asked. Then, the mean values thereof were calculated at each sample.

### Example 1

An ethanol solution of jambu extract was diluted with water under the room temperature to prepare a 30 ppm jambu extract aqueous solution (hereinafter, referred to as 'spilanthol aqueous solution') and tests were conducted by using 100 ml thereof as a sample.

The results are shown in Figure 1. In spilanthol condition, tendencies of stimulative effects were shown in all sessions. It was also confirmed that a stimulative effect was observed at least until 10 minutes after intaking the sample. On the other hand, in water condition, no stimulative effect was observed at least until 30 minutes after intaking.

### Example 2

An ethanol solution of Japanese pepper extract was diluted with water under the room temperature to prepare a 75 ppm Japanese pepper extract aqueous solution (hereinafter, referred to as 'sanshool aqueous solution or hydroxy-sanshool aqueous solution') and tests were conducted by using 100 ml thereof as a sample.

The results are shown in Figure 2. In hydroxy-sanshool condition, tendencies of stimulative effect were shown in all sessions. It was confirmed that a stimulative effect was observed at least until 30 minutes after intaking.

### Example 3

An ethanol solution of white pepper oleoresin was diluted with water under the room temperature to prepare a 50 ppm white pepper oleoresin aqueous solution (hereinafter, referred to as 'piperine aqueous solution') and tests were conducted by using 100 ml thereof as a sample.

The results are shown in Figure 3. In piperine aqueous condition, tendencies of stimulative effect were shown in all sessions. It was confirmed that a stimulative effect was observed at least until 30 minutes after intaking.

Hereinafter, examples of the formulation of stimulating agent or stimulating composition of the present invention included in flavor of fragrance formulated to typical products used for sensory evaluation are listed.

### Example 4

A flavor composition having following ingredients and blending amounts (% by mass) was prepared as a flavor composition to test a stimulative effect.

### [Flavor composition for stimulative gum (cola flavor)]

| Ingredient | Amount (% by mass) |
|---|---|
| Lemon oil cold pressed | 30.0 % |
| Lime oil distilled | 30.0 % |
| Orange oil cold pressed | 2.5 % |
| Cassia oil | 0.5 % |
| Nutmeg oil | 0.2 % |
| Clove oil | 0.2 % |
| Cardamom oil | 0.1 % |
| Coriander oil | 0.1 % |
| Vanillin | 0.2 % |
| Spilanthol 0.1% solution | 10.0 % |
| Medium chain triglyceride | 26.2 % |
| Total | 100.0 % |

Next, a tablet gum composition having following ingredients and blending amounts (% by mass) was prepared.

| Ingredient | Amount (% by mass) |
|---|---|
| Xylitol | 15.0 % |
| Maltitol | 48.8 % |
| Gum base (Regular) | 28.0 % |
| Maltitol syrup (BRIX 75) | 5.0 % |
| Glycerol | 3.0 % |
| Acesulfame K | 0.1 % |
| Aspartame | 0.1 % |
| Total | 100.0 % |

Acesulfame K is 6-methyl-1,2,3-oxathiazine-4(3H)-on-2,2-dioxide.

One mass of the aforementioned flavor composition for stimulation was added to 99 masses of the aforementioned tablet gum composition to obtain a tablet gum containing the flavor composition for stimulation by a usual method.

3. 0 g of the obtained gum containing the flavor composition for stimulation were used as a sample and evaluations of stimulative effect were conducted by subjective evaluation of using a questionnaire. As a result, it was confirmed that tendencies of stimulation were observed in all sessions and stimulation effects were observed at least until 20 minutes after intaking the flavor composition for stimulation.

### Example 5

A flavor composition having following ingredients and blending amounts (% by mass) was prepared as a flavor composition to test a stimulative effect.

### [Flavor composition for stimulation hard candy (cola flavor)]

| Ingredient | Amount (% by mass) |
|---|---|
| Lemon oil cold pressed | 25.0 % |
| Lime oil distilled | 25.0 % |
| Orange oil cold pressed | 2.0 % |
| Cassia oil | 0.5 % |
| Nutmeg oil | 0.2 % |
| Clove oil | 0.2 % |
| Cardamom oil | 0.1 % |
| Coriander oil | 0.1 % |
| Vanillin | 0.2 % |
| Spilanthol 0.1% solution | 2.0 % |
| Medium chain triglyceride | 44.7 % |
| Total | 100.0 % |

Next, a raw material of candy having following ingredients and blending amounts was prepared.

| Ingredient | Amount |
|---|---|
| Granulated sugar | 560.0 g |
| Starch syrup (47 DE, BRIX 85) | 500.0 g |
| Water | 160.0 g |
| Citric acid | 12.0 g |
| Food color | suitable quantity |

Granulated sugar, starch syrup, and water were added to a vessel and heated to dissolve completely. After heated to 150 °C, it was cooled down to 120 °C and citric acid, a food color, and the aforementioned flavor composition were added thereto and mixed thoroughly. The amount of the flavor composition compounded was 0.2 % by mass relative to the total mass of the hard candy. Then, it was moved on a cooling plate and cut and shaped after becoming to appropriate temperature to obtain candies containing the flavor composition for stimulation.

4.0 g of the thus obtained candy containing the flavor composition for stimulation were used as a sample. Evaluations of stimulative effect were conducted by subjective evaluation using a questionnaire. As a result, it was confirmed that tendencies of stimulation were observed in all sessions and the effect was remained at least until 20 minutes after intaking the flavor composition for stimulation.

### Example 6

A flavor composition having following ingredients and blending amounts (% by mass) was prepared as a flavor composition to test a stimulative effect.

### [Flavor composition for energy drink]

| Ingredient | Amount (% by mass) |
|---|---|
| Orange essence | 40.0 % |
| Lime essence | 20.0 % |
| Peach base | 0.1 % |
| Pineapple base | 0.4 % |
| Spilanthol 0.1% solution | 2.0 % |
| 95% alcohol | 22.9 % |
| Deionized water | 14.6 % |
| Total | 100.0 % |

Next, 0.5 ml of the flavor composition for psychic stimulation described above was added to 500 ml of carbonated water to obtain a drink containing the flavor composition for stimulation.

300 ml of the thus obtained drink containing the flavor composition for stimulation were used as a sample. Evaluations of stimulative effect were conducted by subjective evaluation using a questionnaire. As a result, it was confirmed that tendencies of stimulation were observed in all sessions and the effects were remained at least until 20 minutes after intaking the flavor composition for stimulation.

### Example 7

A flavor composition having following ingredients and blending amounts (% by mass) was prepared as a flavor composition to test a stimulative effect.

### [Flavor composition for stimulation dentifrice agent (peppermint type)]

| Ingredient | Amount (% by Mass) |
|---|---|
| L-menthol | 40 % |
| Peppermint oil | 30 % |
| Anethole | 5 % |
| Eugenol | 1 % |
| Eucalyptus oil | 3 % |
| Lemon oil | 2 % |
| Spilanthol | 1 % |
| Ethyl alcohol | 18 % |
| Total | 100 % |

1.0 % by mass of the aforementioned flavor composition for dentifrice agent was added to the following toothpaste base to obtain a raw material for toothpaste. The raw material was degassed while mixing for 10 minutes under 100 mmHg in a mixer and taken it out of the mixer. Then, tubes were individually filled with the mixture in an amount of 15 g per one tube and sealed.

### [Formulation of toothpaste base]

| Ingredient | Amount (% by weight) |
|---|---|
| Calcium bicarbonate | 50.00 |
| Glycerol | 25.00 |
| Purified water | 21.80 |
| Carboxymethyl cellulose | 1.50 |
| Sodium lauryl sulfate | 1.40 |
| Saccharin sodium | 0.25 |
| Sodium benzoate | 0.05 |
| Total | 100.00 |

The toothpaste packed into a tube was kept in a thermostatic chamber at 50 °C for 2 weeks. After that, the toothpaste was taken out of the chamber and cooled down to the room temperature. This toothpaste was used as a sample. After brushing the teeth with a toothbrush on which the toothpaste was placed, evaluations of stimulative effect just after brushing and at every determined time were conducted by subjective evaluation using a questionnaire. As a result, it was confirmed that tendencies of stimulation were observed in all sessions and the effect was remained at least until 20 minutes after intaking the flavor composition for stimulation.

### Example 8

A flavor composition having following ingredients and blending amounts (% by mass) was prepared as a flavor composition for stimulation.

### [Flavor composition for stimulation mouthwash or liquid toothpaste (peppermint type)]

| Ingredient | Amount (% by mass) |
|---|---|
| L-menthol | 25 % |
| Peppermint oil | 45 % |
| Anethole | 1 % |
| Eugenol | 1 % |
| Eucalyptus oil | 3 % |
| Methyl salicylate | 1 % |
| Lemon oil | 1 % |
| Spilanthol | 0.5 % |
| Ethyl alcohol | 22.5 % |
| Total | 100.0 % |

0.1 % by weight of the aforementioned mouthwash flavor (peppermint type) was added to a mouthwash base having the following formulation to obtain a mouthwash. 100 ml of the mouthwash were put into a transparent glass bottle and sealed.

### [Mouthwash base formulation]

| Ingredient | Amount (% by weight) |
|---|---|
| 95% ethyl alcohol | 10.00 |
| Polyoxyethylene hydrogenated | |
| castor oil (EO-60) | 2.00 |
| Concentrated glycerol | 10.00 |
| Saccharin sodium | 0.01 |
| Sodium benzoate | 0.05 |
| Monosodium phosphate monohydrate | 0.06 |
| Sodium hydroxide | suitable quantity (Adjustment to pH 7.8) |
| Purified water | suitable quantity |
| Total | 100.00 |

The mouthwash was stored in the same manner as Example 7 and taken out after 2 weeks. After the temperature of the mouthwash was returned to the room temperature, the mouthwash was used as a sample. 10 to 15 ml of the content was poured into each cup and the mouths of the test subjects were rinsed with the mouthwash. After rinsing, evaluations of stimulative effect just after brushing and at every determined time were conducted by subjective evaluation using a questionnaire. As a result, it was confirmed that tendencies of stimulation were observed in all sessions and the effect was remained at least until 20 minutes after intaking the flavor composition for stimulation.

### Example 9

3 cm X 3 cm square absorbent cottons impregnated with 0.3 g of an aqueous solution (spilanthol 1% ethanol solution : water = 3 : 7) were prepared as a sample.

Ten healthy adults were adopted as test subjects and the absorbent cottons impregnated spilanthol were attached on their left arms. Evaluations of stimulative effect during attachment of the adsorbent cotton were conducted by subjective evaluation of sleepiness using a questionnaire. As a result, sleepiness of 5 subjects lowered remarkably at 15 minutes after sample attachment and there is no subject whose sleepiness increased with time. Therefore, it was confirmed that stimulative effects were remained at least until 15 minutes after sample attachment.

## Claims

1. A stimulating agent consisting of a pungent component having astringency.

2. The stimulating agent according to claim 1, wherein the pungent component having astringency is an extract of one or two or more of plants belonging to a genus selected from a group consisting of the genus Spilanthes of family Asteraceae, the genus Zanthoxylum of family Rutaceae, the genus Fagara of family Rutaceae, and the genus Piper of family Piperaceae.

3. The stimulating agent according to claim 1, wherein the pungent component having astringency is an amide derivative represented by the following formula (1): 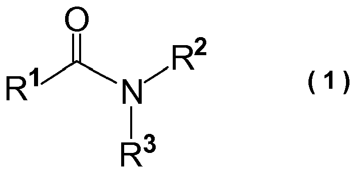 wherein R¹ represents an alkenyl group having 2 to 12 carbon atoms which may be substituted by a methylenedioxyphenyl group at the end, R² and R³ may be the same or different from each other and each represents a hydrogen atom or a lower alkyl group which may be substituted by a hydroxyl group, and R² and R³ may form a heterocyclic ring together with a nitrogen atom adj acent thereto.

4. The stimulating agent according to claim 3, wherein the amide derivative represented by the formula (1) is one or two or more of amide derivatives selected from a group consisting of spilanthols, sanshools, hydroxy-sanshools, and piperines.

5. A stimulating composition containing a pungent component having astringency as effective ingredients.

6. The stimulating composition according to claim 5, wherein the pungent component having astringency is an extract of one or two or more of plants belonging to a genus selected from a group consisting of the genus Spilanthes of family Asteraceae, the genus Zanthoxylum of family Rutaceae, the genus Fagara of family Rutaceae, and the genus Piper of family Piperaceae.

7. The stimulating composition according to claim 5, wherein the pungent component having astringency is an amide derivative represented by the formula (1): 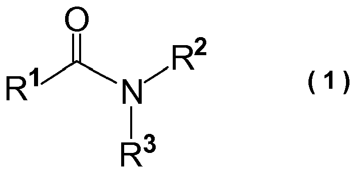 wherein R¹ represents an alkenyl group having 2 to 12 carbon atoms which may be substituted by a methylenedioxyphenyl group at the end, R² and R³ may be the same or different from each other and each represents a hydrogen atom or a lower alkyl group which may be substituted by a hydroxyl group, and R² and R³ may form a heterocyclic ring together with a nitrogen atom adj acent thereto.

8. The stimulating composition according to claim 7, wherein the amide derivative represented by the formula (1) is one or two or more of amide derivatives selected from a group consisting of spilanthols, sanshools, hydroxy-sanshools, and piperines.

9. The stimulating composition according to any one of claims 5 to 8, wherein the pungent component having astringency is contained in 0.000001 to 10.0 % by mass.

10. A flavor or fragrance composition wherein the stimulating agent according to any one of claims 1 to 4 or the stimulating composition according to any one of claims 5 to 9 is contained.

11. A food, a drink, an oral composition or an external skin preparation wherein the stimulating agent according to any one of claims 1 to 4 or the stimulating composition according to any one of claims 5 to 9 is contained.

12. A food, a drink, an oral composition or an external skin preparation wherein the flavor or fragrance composition according to claim 10 is contained.

13. Use of a pungent component having astringency as a stimulating agent.

14. Use according to claim 13 wherein the pungent component having astringency is an extract of one or two or more of plants belonging to a genus selected from a group consisting of the genus Spilanthes of family Asteraceae, the genus Zanthoxylum of family Rutaceae, the genus Fagara of family Rutaceae, and the genus Piper of family Piperaceae.

15. Use according to claim 13 wherein the pungent component having astringency is an amide derivative represented by the following formula (1): 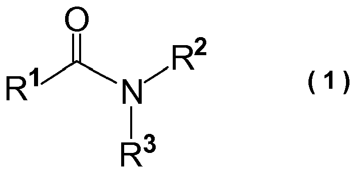 wherein R¹ represents an alkenyl group having 2 to 12 carbon atoms which may be substituted by a methylenedioxyphenyl group at the end, R² and R³ may be the same or different from each other and each represents a hydrogen atom or a lower alkyl group which may be substituted by a hydroxyl group, and R² and R³ may form a heterocyclic ring together with a nitrogen atom adj acent thereto.

16. Use according to claim 15 wherein the amide derivative represented by the formula (1) is one or two or more of amide derivatives selected from a group consisting of spilanthols, sanshools, hydroxy-sanshools, and piperines.
